(19) Europäisches Patentamt
European Patent Office
Office européen des brevets

(11) Veröffentlichungsnummer: **0 153 617**
**B1**

(12) # EUROPÄISCHE PATENTSCHRIFT

(45) Veröffentlichungstag der Patentschrift: **20.04.88**

(51) Int. Cl.⁴: **C 07 D 413/12**, C 07 D 417/12, A 61 K 31/53, A 23 K 1/16

(21) Anmeldenummer: **85101174.2**

(22) Anmeldetag: **05.02.85**

(54) **1-[4-(Benzothia- oder -oxazol-2-ylthio- oder -2-yloxy) phenyl]-1,3,5-triazin-2,4,6(1H,3H,5H)-trione, Verfahren zu ihrer Herstellung und ihre Verwendung als Arzneimittel.**

(30) Priorität: **15.02.84 DE 3405241**

(43) Veröffentlichungstag der Anmeldung: **04.09.85 Patentblatt 85/36**

(45) Bekanntmachung des Hinweises auf die Patenterteilung: **20.04.88 Patentblatt 88/16**

(84) Benannte Vertragsstaaten: **AT BE CH DE FR GB IT LI NL SE**

(56) Entgegenhaltungen:
**GB - A - 998 368**
**US - A - 2 733 243**

**CHEMICAL ABSTRACTS, Band 86, No. 15, 11. April 1977, Columbus, Ohio, USA. ETIENNE, ANDRE; LONCHAMBON, GEORGES; ROQUES, JACQUES. "Mixed N-phenyl and N-dialkyl isocyanurates." Seite 507, Spalte 1, Zusammenfassung-Nr. 106 534t**

(73) Patentinhaber: **BAYER AG, Konzernverwaltung RP Patentabteilung, D-5090 Leverkusen 1 Bayerwerk (DE)**

(72) Erfinder: **Haberkorn, Axel, Dr., Fuhlrottstrasse 99, D-5600 Wuppertal 1 (DE)**
Erfinder: **Kölling, Heinrich, Dr., Adlerstrasse 15, D-5657 Haan (DE)**
Erfinder: **Kume, Toyohiko, 6-7-8, Asahigaoka, Hino-shi Tokyo (JP)**
Erfinder: **Kuyama, Shinpei, Dr., 3-21-39, Meguro-Ku, Tokyo (JP)**

**EP 0 153 617 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 1-[4-(Benzothia- oder -oxazol-2-ylthio- oder -2-yloxy)-phenyl]-1,3,5-triazin-2,4,6-(1H, 3H, 5H)-trione, Verfahren zu ihrer Herstellung und ihre Verwendung als Arzneimittel, insbesondere als anticoccidiale Mittel für den Menschen und für verschiedene Tierarten.

Es ist bereits bekannt geworden, dass α-(4-Phenylthiophenyl)-1,2,4-triazin-3,5-dione eine Wirkung zur Bekämpfung von Coccidiose haben. Die Wirkung befriedigt jedoch vor allem bei niedrigen Aufwandkonzentrationen nicht in allen Fällen.

Es wurden die neuen 1-[4-(Benzothia- oder -oxazol-2-ylthio- oder -2-yloxy)phenyl]-1,3,5-triazin-2,4,6(1H, 3H, 5H)-trione der Formel (I)

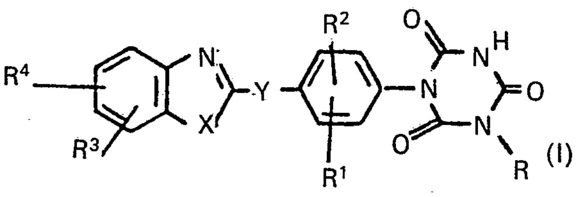 (I)

in der

R für $C_{1-4}$-Alkyl steht,

$R^1$ und $R^2$ gleich oder voneinander verschieden sind und für Wasserstoff, Halogen, $C_{1-4}$-Alkyl stehen,

$R^3$ für Halogen, $C_{1-4}$-Alkyl, $C_{1-4}$-Alkoxy, $C_{1-4}$-Alkylthio, $C_{1-4}$-Halogenalkyl, $C_{1-4}$-Halogenalkoxy oder $C_{1-4}$-Halogenalkylthio steht und

$R^4$ für Wasserstoff oder Halogen steht,

X und Y gleich oder voneinander verschieden sind und für Schwefel oder Sauerstoff stehen,

sowie ihre Salze gefunden.

Die Verbindungen der Formel I und ihre Salze können hergestellt werden durch Umsetzung eines substituierten Harnstoffs der Formel

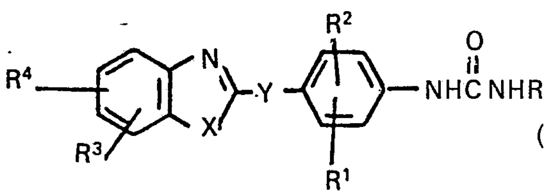 (II)

in der

R, $R^1$, $R^2$, $R^3$, $R^4$, X und Y die oben angegebene Bedeutung haben,

mit einem Carbonylisocyanat der Formel

$$Z-\overset{\overset{O}{\|}}{C}-N=C=O \qquad (III)$$

in der

Z für Halogen, Alkoxy oder Aryloxy steht,

und im Falle der Salze, durch Umsetzung einer Verbindung der Formel I mit einer Base.

Die substituierten Harnstoffe der Formel II sind neu. Sie können hergestellt werden,

a) durch Umsetzung eines niederen Alkylisocyanats mit einem Amin der Formel IV

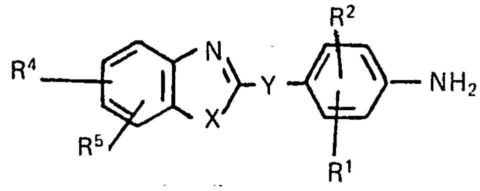 (IV)

in der

$R^1$, $R^2$, $R^3$, $R^4$, X und Y die im Vorstehenden angegebenen Bedeutungen haben,

oder

b) durch Umsetzung eines niederen Alkylamins mit einem Isocyanat der Formel V

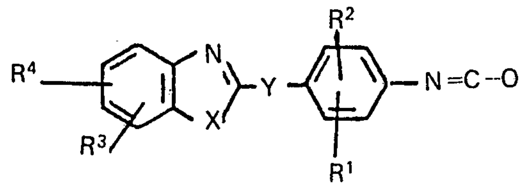 (V)

in der

$R^1$, $R^2$, $R^3$, $R^4$, X und Y die im Vorstehenden angegebenen Bedeutungen haben,

oder

c) durch Umsetzung eines Harnstoffs der Formel VI

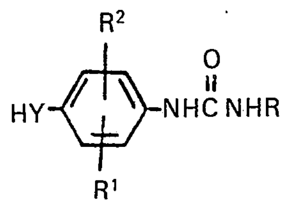 (VI)

in der

R, $R^1$, $R^2$ und Y die im Vorstehenden angegebenen Bedeutungen haben,

mit einer Verbindung der Formel VII

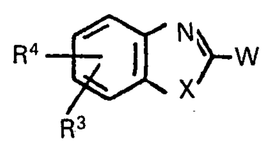

in der

$R^3$, $R^4$ und X die im Vorstehenden angegebenen Bedeutungen haben und

W Chlor oder Brom bezeichnet,

in Gegenwart einer geeigneten Base.

Verbindungen der Formel IV sind zum Teil bekannt (Chemical Abstracts CA 93-71 689; 72-134 130; 78-58 423). Neue Verbindungen der Formel IV lassen sich nach an sich bekannten Methoden herstellen (DE-A-2 225 071).

Verbindungen der Formel V sind neu. Sie werden hergestellt, indem man z.B. Verbindungen der Formel IV in an sich bekannter Weise mit Phosgen oder Trichlormethoxycarbonylchlorid umsetzt.

Verbindungen der Formel VI sind bekannt oder lassen sich nach an sich bekannten Methoden herstellen (J. Med. Chem. 1977, 20(5), S. 705–708, Chemical Abstracts CA 96-6442; 91-193 023; 87-78 230).

Verbindungen der Formel VII sind bekannt oder lassen sich nach an sich bekannten Methoden herstellen (J.O.C. 19, 758; EP-A-43 573).

Bevorzugt sind Verbindungen der Formel I, in welcher

R für $C_{1-4}$-Alkyl steht,

$R^1$ und $R^2$ für Wasserstoff, Chlor oder $C_{1-4}$-Alkyl, in 3'- und 5'-Stellung stehen,

$R^3$ für Halogen, $C_{1-4}$-Alkyl, $C_{1-4}$-Alkoxy, $C_{1-4}$-Halogenalkoxy, $C_{1-4}$-Halogenalkyl, $C_{1-4}$-Alkylthio, $C_{1-4}$-Halogenalkylthio steht,

$R^4$ für Wasserstoff oder Halogen, insbesondere Chlor steht,

X für O oder S steht,

Y für O oder S steht.

Insbesondere seien genannt Verbindungen der Formel I, in welcher

R für Methyl, Ethyl oder Propyl steht,

$R^1$ und $R^2$ für Wasserstoff, Chlor oder Methyl, insbesondere in 3'- oder 5'-Stellung stehen,

$R^3$ für Fluor, Chlor, Brom, Jod, Methyl, Ethyl, Methoxy, Ethoxy, Methylthio, Trifluormethyl, Trifluormethoxy, Trifluormethylmercapto steht,

$R^4$ für Wasserstoff oder Chlor steht,

X und Y für O oder S stehen.

Die Verbindungen der Formel I und ihre Salze lassen sich vorteilhaft zur Bekämpfung von Coccidiosen bei Mensch und Tier, insbesondere bei Geflügel und Haustieren einsetzen. Neben ihrer ausgezeichneten Wirkung ist ihr rascher Abbau im Organismus zu erwähnen.

Die Umsetzung der Verbindungen der Formel II mit einem Carbonylisocyanat der Formel III lässt sich durch folgendes Formelschema wiedergeben:

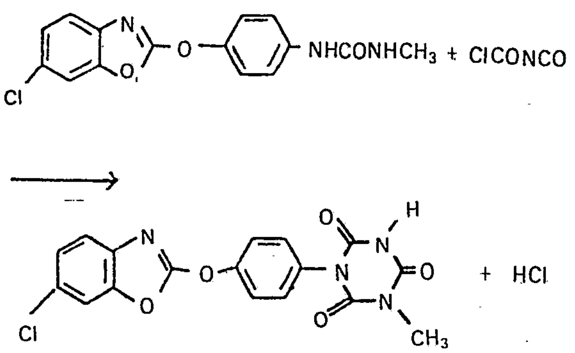

Als Verbindungen der Formel II seien bevorzugt genannt solche, in denen

R für $C_{1-4}$-Alkyl insbesondere Methyl, Ethyl oder Propyl steht,

$R^1$ und $R^2$ für Wasserstoff, Chlor oder Methyl stehen, wobei die Substituenten, insbesondere in 5'- und 3'-Stellung stehen,

$R^3$ für Fluor, Chlor, Brom, Jod, $C_{1-4}$-Alkyl, insbesondere Methyl, $C_{1-4}$-Alkoxy, insbesondere Methoxy, Ethoxy, $C_{1-4}$-Halogenalkoxy, insbesondere Trifluormethoxy, $C_{1-4}$-Halogenalkyl, insbesondere Trifluormethyl, $C_{1-4}$-Alkylmercapto, insbesondere Methylmercapto, $C_{1-4}$-Halogenalkylmercapto, insbesondere Trifluormethylmercapto steht,

$R^4$ für Wasserstoff oder Halogen, insbesondere Chlor steht,

X für Sauerstoff oder Schwefel steht und

Y für Sauerstoff oder Schwefel steht.

Als Carbonylisocyanat sei bevorzugt Chlorcarbonylisocyanat genannt.

Die Reaktion wird in inerten organischen Lösungsmitteln durchgeführt. Dazu gehören Kohlenwasserstoffe wie insbesondere Benzol, Toluol, Xylol, halogenierte Kohlenwasserstoffe wie Chlorbenzol, Dichlorbenzol, Methylenchlorid, Chloroform, Ether wie Tetrahydrofuran, Dioxan.

Die Reaktion wird üblicherweise bei Normaldruck und bei einer Temperatur zwischen 0 und 150 °C, insbesondere bei 80–120 °C durchgeführt.

Die Verbindungen werden in etwa in äquimolaren Mengen eingesetzt. Es kann von Vorteil sein, das Carbonylisocyanat in Überschuss einzusetzen.

Die Salzbildung erfolgt, indem man die Verbindungen der Formel I mit einer anorganischen oder organischen Base umsetzt. Es werden dazu Basen eingesetzt, die zu Salzen führen, die in den bei Coccidiosemitteln üblichen Konzentrationen für den Warmblüterorganismus unschädlich sind.

Als Basen seien genannt: z.B. Kaliumhydroxid, Natriumhydroxid, Ammoniak, Methylamin.

Verbindungen der Formel II sind neu.

Ihre Herstellung aus Verbindungen der Formel IV und Alkylisocyanaten lässt sich durch folgendes Formelschema wiedergeben:

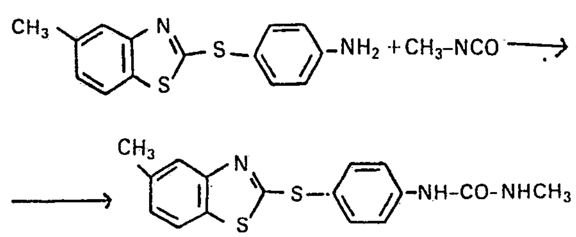

Bevorzugt werden Verbindungen der Formel IV eingesetzt, in welcher $R^1$, $R^2$, $R^3$, $R^4$ die weiter oben angegebene bevorzugte Bedeutung haben.

Als Alkylisocyanate seien bevorzugt genannt: Methyl-, Ethyl-, Propylisocyanat.

Die Umsetzung erfolgt im allgemeinen in Gegenwart eines inerten Verdünnungsmittels. Als bevorzugte Verdünnungsmittel seien die beim Verfahren zur Herstellung der Verbindungen der Formel I genannten erwähnt.

Die Umsetzung erfolgt bei Temperaturen von 0–150 °C, insbesondere bei 20–80 °C.

Man arbeitet bevorzugt bei Normaldruck.

Die Verbindungen werden in etwa äquimolaren Mengen eingesetzt. Von Vorteil kann ein Überschuss des Isocyanats sein.

Verbindungen der Formel II werden auch erhalten, indem man Verbindungen der Formel V mit Alkylaminen umsetzt. Die Reaktion lässt sich durch folgendes Formelschema wiedergeben:

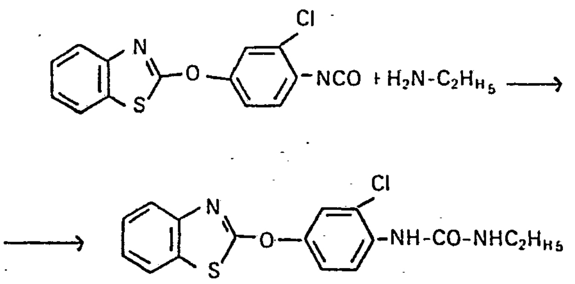

Bevorzugte Verbindungen der Formel V sind die in denen $R^1$, $R^2$, $R^3$, $R^4$ die weiter oben als bevorzugt angegebenen Bedeutungen haben.

Die Umsetzung erfolgt in an sich bekannter Weise, z.B. in den weiter oben angegebenen Verdünnungsmitteln. Auch die Reaktionsbedingungen können wie weiter oben angegeben, gewählt werden.

Verbindungen der Formel II werden auch erhalten, indem man Verbindungen der Formel VI mit Verbindungen der Formel VII umsetzt. Die Reaktion lässt sich durch folgendes Formelschema wiedergeben:

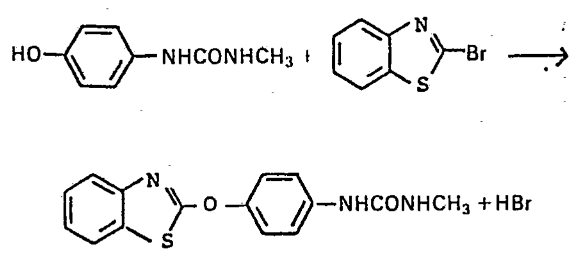

Bevorzugte Verbindungen der Formel VI sind die, in denen R, $R^1$, $R^2$, Y die weiter oben als bevorzugt angegebenen Bedeutungen haben.

Bevorzugte Verbindungen der Formel VII sind die in denen $R^3$, $R^4$ die weiter oben als bevorzugt angegebenen Bedeutungen haben.

Die Umsetzung erfolgt z.B. in einem der oben angegebenen Verdünnungsmittel. Die Umsetzung erfolgt in Gegenwart einer Base. Als solche seien genannt anorganische und organische Basen wie z.B. KOH, NaOH, $Ca(OH)_2$, Pyridin, Triethylamin.

Die Verbindungen der Formeln VI und VII sowie die Base werden in etwa in äquimolaren Mengen zusammengegeben. Ein geringer Überschuss der einen oder anderen Komponente ist für die Ausbeute unwesentlich.

Die erfindungsgemässen Verbindungen und ihre Salze weisen gute Aktivität gegen Coccidien bei Geflügel, z.B. Eimeria tenella (Blinddarm-Coccidiose bei Hühnern), E. acervulina, E. brunetti, E. maxima, E. mitis, E. mivati, E. necatrix und E. praecox (Coccidiose des Dünndarms bei Hühnern), auf.

Die Zubereitungen können ausserdem zur Prophylaxe und Behandlung von Coccidiose-Infektionen anderer Arten von Hausgeflügel verwendet werden. Darüber hinaus weisen die erfindungsmässen Verbindungen starke Aktivität bei Coccidieninfektionen von Säugetieren auf. Ferner können die Verbindungen bei der Behandlung oder Prophylaxe der Toxoplasmose sowohl für die Behandlung von Katzen, die für die Ausscheidung der infektiösen Stadien (Oozysten) verantwortlich sein können, und für die Behandlung von infizierten Menschen verwendet werden. Die neuen Verbindungen können auch zur Behandlung und Prophylaxe von Sarcocystis-Infektionen, sowohl in dem obligaten Zwischenwirt (z.B. Schwein, Rind, Schaf) als auch in dem definitiven Wirt (z.B. Mensch, Katze, Hund), verwendet werden.

Coccidieninfektionen können zu schweren Verlusten unter den Haustieren führen und stellen ein wirkliches Problem bei der Aufzucht von Geflügel und Säugetieren wie Rindvieh, Schaf, Kaninchen und Hunden, dar. Die Wirkung der bekannten Mittel gegen Coccidiose ist in den meisten Fällen auf einige wenige Arten von Geflügel begrenzt. Die Behandlung und Prophylaxe der Coccidiose bei Säugetieren stellt bisher ein grosses ungelöstes Problem dar.

Die Arzneimittelzubereitungen gemäss der Erfindung enthalten eine grössere oder geringere Menge, z.B. 0,1 % bis 99,5 %, vorzugsweise 0,5 % bis 90 %, wenigstens einer erfindungsgemässen Verbindung der Formel I oder ihrer Salze in Kombination mit einem pharmazeutisch unbedenklichen, ungiftigen inerten Streckmittel oder Träger, wobei der Träger ein oder mehrere feste, halbfeste oder flüssige Verdünnungs- oder Streckmittel, Füllstoffe und Zubereitungsadjuvantien enthält, die ungiftig, inert und pharmazeutisch unbedenklich sind. Diese Arzneimittelzubereitungen liegen vorzugsweise in Form von Dosierungseinheiten, d.h. physikalisch gesonderten Einheiten, vor, die eine vorbestimmte Menge des Medikaments enthalten, die einem Bruchteil oder einem Vielfachen der Dosis entspricht, die laut Berechnung die gewünschte therapeutische Wirkung hervorbringt. Die Dosierungseinheiten können eine, zwei, drei, vier oder mehr Einzeldosen oder als Alternative eine Hälfte, ein Drittel oder Viertel einer Einzeldosis enthalten. Eine Einzeldosis enthält vorzugsweise eine Menge, die genügt, um die gewünschte therapeutische Wirkung nach der Verabreichung bei einmaliger Anwendung einer oder mehrerer Dosierungseinheiten gemäss einer vorbestimmten Dosierungsbehandlungsvorschrift, gewöhnlich einer ganzen, halben, einem Drittel oder Viertel der einmal, zweimal, dreimal oder viermal täglich verabreichten Tagesdosis hervorzubringen. Andere therapeutische wirksame Substanzen können ebenfalls enthalten sein.

Die orale Verabreichung kann unter Verwendung von festen und flüssigen Dosierungseinheitsformen, z.B. Pulver, Tabletten, Dragees, Kapseln, Granulat, Suspensionen, Lösungen und dergleichen, erfolgen.

Pulver werden hergestellt durch Mahlen der Verbindungen auf eine geeignete feine Grösse und Vermischen mit einem in ähnlicher Weise gemahlenen pharmazeutischen Träger, wie einem

essbaren Kohlenhydrat, z.B. Stärke, Lactose, Saccharose, Glucose oder Mannit. Süssungsmittel, Aromatisierungsmittel, Konservierungsmittel, Dispergierungs- und Färbemittel können ebenfalls vorhanden sein.

Kapseln werden hergestellt durch Zubereitung eines Pulvergemisches, wie es vorstehend beschrieben wurde, und Einfüllen in geformte Gelatinehüllen. Gleitmittel und Schmiermittel, z.B. kolloidales Siliciumdioxyd, Talkum, Magnesiumstearat, Calciumstearat oder festes Polyethylenglykol, können dem Pulvergemisch vor dem Füllvorgang zugesetzt werden. Ein Sprengmittel oder löslich machendes Mittel, z.B. Agar-Agar, Calciumcarbonat oder Natriumcarbonat, können ebenfalls zugesetzt werden, um die Verfügbarkeit des Medikaments zu verbessern, wenn die Kapsel eingenommen wird.

Tabletten werden beispielsweise formuliert durch Herstellung eines Pulvergemisches, Granulieren oder Verperlen, Zusetzen eines Gleitmittels und Sprengmittels und Pressen zu Tabletten. Ein Pulvergemisch wird hergestellt durch Mischen der in geeigneter Weise zerkleinerten Verbindung mit einem Verdünnungs- oder Streckmittel oder einer Base in der vorstehend beschriebenen Weise und, falls gewünscht, mit einem Bindemittel wie Carboxymethylcellulose, einem Alginat, Gelatine oder Polyvinylpyrrolidon, einem Lösungsverzögerer, z.B. Paraffin, einem Resorptionsbeschleuniger, z.B. einem quaternären Salz, und/oder einem Absorptionsmittel wie Bentonit, Kaolin oder Dicalciumphosphat. Das Pulvergemisch kann granuliert werden durch Benetzen mit einem Bindemittel wie Sirup, Stärkepaste, Schleim von Gummi arabicum oder Lösungen von Cellulosematerialien oder polymeren Materialien, und Pressen durch ein Sieb. Als Alternative zu dem Granulieren kann das Pulvergemisch durch die Tablettiermaschine gegeben werden, und die hierbei erhaltenen unvollkommen geformten Perlen können zu Granulat zerbrochen werden. Das Granulat kann zur Verhinderung des Anklebens an die Tablettenbildungsdüsen durch Zusatz von Stearinsäure, einem Stearatsalz, Talkum oder Mineralöl geschmiert werden. Das geschmierte Gemisch wird dann zu Tabletten gepresst. Die Medikamente können auch mit rieselfähigen inerten Trägern kombiniert und direkt zu Tabletten gepresst werden, ohne dass sie die Granulier- oder Verperlungsstufen durchlaufen. Ein klarer oder undurchsichtiger Schutzüberzug, bestehend aus einem abdichtenden Überzug aus Schellack, ein Überzug aus Zucker oder polymeren Material und ein polierender Wachsüberzug können vorgesehen werden. Diesen Überzügen können Farbstoffe zur Unterscheidung verschiedener Dosierungseinheiten zugesetzt werden.

Orale Flüssigkeiten, z.B. Lösungen, Sirupe und Elixiere, können in Dosierungseinheitsform so hergestellt werden, dass eine gegebene Menge eine vorbestimmte Menge der Verbindung enthält. Sirupe können durch Auflösen der Verbindung in einer in geeigneter Weise schmackhaft gemachten wässrigen Saccharoselösung hergestellt werden, während Elixiere durch Verwendung eines ungiftigen alkoholischen Trägers hergestellt werden. Suspensionen können durch Dispergieren der Verbindung in einem ungiftigen Träger hergestellt werden. Löslichmachende Mittel und Emulgatoren, z.B. ethoxylierte Isostearylalkohole und Polyoxyethylensorbitester, Konservierungsmittel, Geschmack verleihende Zusatzstoffe, z.B. Pfefferminzöl oder Saccharin und dergleichen können ebenfalls zugesetzt werden.

Falls angemessen, können Dosierungseinheitszubereitungen für die orale Verabreichung in Form von Mikrokapseln hergestellt werden. Die Zubereitung kann auch so hergestellt werden, dass die Freigabe verlängert oder in Gang gehalten wird, beispielsweise durch Umhüllen oder Einbetten des feinteiligen Materials in Polymerisaten, Wachs oder dergleichen.

Die parenterale Verabreichung kann unter Verwendung von flüssigen Dosierungseinheitsformen, z.B. sterilen Lösungen und Suspensionen, die für die subkutane, intramuskuläre oder intravenöse Injektion vorgesehen sind, erfolgen. Diese Zubereitungen werden hergestellt durch Suspendieren oder Auflösen einer gemessenen Menge der Verbindung in einem für die Injektion geeigneten ungiftigen flüssigen Träger, z.B. einem wässrigen oder öligen Medium, und Sterilisation der Suspension oder Lösung. Als Alternative wird eine abgemessene Menge der Verbindung in eine Ampulle gegeben, und die Ampulle und ihr Inhalt werden sterilisiert und zugeschmolzen. Eine zugehörige Ampulle oder ein zugehöriger Träger können zum Vermischen vor der Verabreichung vorgesehen werden. Ungiftige Salze und Salzlösungen können zugesetzt werden, um die Injektionslösung isotonisch zu machen. Stabilisatoren, Konservierungsmittel und Emulgatoren können ebenfalls zugesetzt werden.

Die rektale Verarbeitung kann unter Verwendung von Suppositorien wirksam sein, in denen die Verbindung mit niedrigschmelzenden, wasserlöslichen oder unlöslichen Feststoffen, z.B. Polyethylenglykol, Kakaobutter, höheren Estern, z.B. Myristylpalmitat oder deren Gemische gemischt ist.

Die topische Anwendung kann unter Verwendung von festen Dosierungseinheitsformen, z.B. Pulvern, oder flüssigen oder halbflüssigen Dosierungseinheitsformen, z.B. Lösungen, Suspensionen, Salben, Pasten, Cremes und Gelen erfolgen. Die Pulver werden unter Verwendung von Trägern wie Talkum, Bentonit, Kieselsäure, Polyamidpulver und dergleichen formuliert. Den flüssigen und halbflüssigen Zubereitungen können Träger zusätzlich zu den vorstehend beschriebenen, z.B. Polyethylenglykol, Pflanzen- und Mineralöle, Alkohole, z.B. Isopropanol, zugesetzt werden. Andere Hilfsstoffe, z.B. Emulgatoren, Konservierungsmittel, färbende Mittel, Duftstoffe und dergleichen, können ebenfalls anwesend sein. Die Zubereitungen können auch als Aerosole verabreicht werden, wobei die üblichen Treibmittel, z.B. die Chlorfluorkohlenwasserstoffe, verwendet werden.

Die bevorzugte Tagesdosis beträgt 25 mg bis 25 g des aktiven Inhaltsstoffes.

Während zu den Darreichungswegen die orale, parenterale (d.h. intramuskuläre, intraperitoneale und intravenöse) rektale und topische Verabreichung gehört, wird die orale Verabreichung besonders bevorzugt.

Die Erfindung umfasst ferner Futter- und Nahrungsmittel, die 5 bis 5000 ppm, vorzugsweise 50 bis 250 ppm einer Verbindung gemäss der Erfindung in Kombination mit einem geeigneten geniessbaren Material, z.B. mit dem in der folgenden Formulierung beschriebenen Hühnerfuttermittel, enthalten:

52 000 % geschrotetes Futtergetreide
17 995 % geschrotete Sojabohnen
5 000 % Maisgluten-Futtermittel
5 000 % Vollweizenmehl
3 000 % Fischmehl
3 000 % Tapiokamehl
3 000 % Mehl der grünen Luzerne
2 000 % gemahlene Weizenkeime
2 000 % Sojaöl
1 600 % Fischgrätenmehl
1 500 % Molkepulver
1 400 % Calciumcarbonat für Futter- und Lebensmittel
1 000 % Calciumphosphat für Futter- und Lebensmittel
1 000 % Melase
0,500 % Bierhefe
0,005 % 1-[4-(6-Chlorobenzothiazol-2-ylthio)-phenyl]-1,3,5-triazin-2,4,6(1H, 3H, 5H)-trion
100 000 %

Ein solches Futter- und Nahrungsmittel kann sowohl für Heilzwecke als auch für prophylaktische Zwecke verwendet werden.

Die vorliegende Erfindung umfasst ferner ein Konzentrat oder eine Vormischung, die 1 bis 30 Gew.-%, vorzugsweise 10 bis 20 Gew.-% einer Verbindung gemäss der Erfindung in Mischung mit einem geniessbaren organischen oder anorganischen Träger enthält, z.B. Maismehl oder Mais- und Sojabohnenmehl oder Mineralsalze, die vorzugsweise eine geringe Menge eines geniessbaren Staubverhütungsöls, z.B. Maisöl oder Sojabohnenöl, enthalten. Die hierbei erhaltene Vormischung kann dann dem vollständigen Geflügel-Futtermittel vor seiner Verfütterung an die Tiere zugesetzt werden.

Die Verbindungen gemäss der Erfindung können auch mit dem Trinkwasser für die Tiere zur Massenbehandlung oder -prophylaxe der Coccidiose gemischt werden.

Das Futtermittel kann sowohl für Heilzwecke als auch für prophylaktische Zwecke verwendet werden.

Für die Heilbehandlung und Prophylaxe der Coccidiose bei Geflügel, insbesondere bei Hühnern, Enten, Gänsen und Truthähnen, werden 25 bis 100 ppm, vorzugsweise 50 bis 100 ppm einer Verbindung gemäss der Erfindung mit einem geeigneten geniessbaren Material, z.B. einem nahrhaften Futtermittel, gemischt. Falls gewünscht, können diese Mengen erhöht werden, besonders wenn die Verbindung vom Empfänger gut vertragen wird.

Für die Behandlung von Einzeltieren, z.B. im Falle der Behandlung der Coccidiose bei Säugetieren oder der Toxoplasmose, werden vorzugsweise Mengen von 5 bis 250 mg/kg Körpergewicht täglich verabreicht, um die gewünschten wirksamen Ergebnisse zu erzielen. Trotzdem kann es zeitweilig notwendig sein, von den genannten Mengen abzuweichen, insbesondere in Abhängigkeit vom Körpergewicht des Versuchstieres oder der Art der Verabreichungsmethode, aber auch wegen der Tierart und seiner individuellen Reaktion auf das Medikament oder der Art der Formulierung und dem Zeitpunkt oder dem Intervall, zu dem es verabreicht wird. So kann es in gewissen Fällen genügen, mit weniger als der vorstehend genannten Mindestmenge auszukommen, während in anderen Fällen die genannte obere Grenze überschritten werden muss. Bei der Verabreichung grösserer Mengen kann es zweckmässig sein, diese im Verlauf des Tages in mehrere Einzeldarreichungen zu unterteilen.

Die coccidiostatische Aktivität von Verbindungen, die für die Verbindungen gemäss der Erfindung repräsentativ sind, wird in Tabelle 1 veranschaulicht, wo eine Eimeria tenella (Blinddarm-Coccidiose/Hühner), Eimeria acervulina und Eimeria maxima (Coccidiose des Dünndarms von Hühnern) als Beispiele der Aktivität im Falle der Coccidiose bei Geflügel genannt sind, während Eimeria falciformis (Coccidiose/Mäuse) als Coccidium bei Säugetieren angegeben ist.

Wenn beispielsweise 9 bis 11 Tage alte Küken mit 4000 sporulierten Oozysten von stark virulenten Stämmen von E. acervulina, E. maxima und E. tenella, den Krankheitserregern der intestinalen Coccidiose, infiziert werden, scheiden die unbehandelten Vergleichstiere täglich 300 000 bis 500 000 Oozysten/g Kot vom fünften bis neunten Tage nach der Infektion aus. Im Verlauf der Krankheit ist die Gewichtszunahme wesentlich verringert, und ernsthafte, makroskopisch nachweisbare pathologische Veränderungen treten hauptsächlich in den Därmen auf, was zu ernsthaften kämorrhagischen Diarrhoeen führen kann, und die Küken können sterben. Bei der Untersuchung der Wirksamkeit gegen E. acervulina, E. maxima und E. tenella wurden die Verbindungen gemäss der Erfindung 3 Tage vor der Infektion bis 8 Tage nach der Infektion (Ende des Versuchs) verabreicht.

Die Zahl der Oozysten wurde mit Hilfe der McMaster-Kammer bestimmt (siehe Engelbrecht et al., «Parasitologische Arbeitsmethoden in Medizin und Vererinärmedizin», S. 172, Akademie-Verlag Berlin (1965) ).

Die Behandlung der Infektion durch Eimeria falciformis bei Mäusen, die als Beispiel der Coccidiose bei Säugetieren erwähnt ist, fand am ersten, zweiten, dritten, sechsten, siebten und achten Tag nach der Infektion statt. Die Infektion erfolgte

mit 10 000 sporulierten Oozysten pro Maus (Gewicht 15 g). Im Falle der unbehandelten Vergleichstiere fanden massive Ausscheidung von Oozysten, bluthaltige Diarrhöe und 30% Sterblichkeit der Tiere, die der Infektion zuzuschreiben waren, vom siebten Tage nach der Infektion an statt.

Als wirksam wurden diejenigen Dosen angesehen, die die Ausscheidung von Oozysten und/ oder klinische Symptome der Coccidiose einschliesslich der Mortalität vollständig oder in hohem Masse verhüteten.

Die niedrigsten wirksamen Dosierungen, die unter den Versuchsbedingungen gefunden wurden, waren niedriger als diejenigen der anticoccidialen Medikamente, die gewöhnlich zur Verhütung der Coccidiose bei Geflügel verwendet werden. Ferner sind die Verbindungen gemäss der Erfindung – ausser einem günstigen Verhalten hinsichtlich der Rückstände in den Kadavern – sehr wirksam gegen die Coccidiose bei Säugetieren (Tab. 1).

Die weit verbreitete Resistenz der Coccidiose gegen weithin verwendete anticoccidiale Mittel erfordert wirksamere Verbindungen, die von neuen Klassen von chemischen Verbindungen abgeleitet sind. Auch von diesem Standpunkt aus bereichern die Verbindungen gemäss der Erfindung in wertvoller Weise die Vielfalt der Medikamente.

Tabelle 1

Vergleich der wirksamen Mindestdosierungen von Verbindungen gemäss der vorliegenden Erfindung mit denjenigen von zwei üblicherweise in der Praxis verwendeten anticoccidialen Mitteln

| Beispiel Nr. | E. acervulina ppm | E. maxima ppm | E. tenella ppm | E. falciformis mg/kg |
|---|---|---|---|---|
| 2 | 25 | 25 | 25 | 1 |
| 9 | 250 | 250 | 250 | 0,5 |
| 11 | 250 | 250 | 250 | 100 |
| 14 | 25 | 25 | 25 | 2,5 |
| 16 | 5 | 5 | 10 | 5 |
| 24 | 100 | 100 | 100 | 25 |
| 26 | 10 | 10 | 10 | 10 |
| 43 | 50 | 50 | 50 | 5 |
| 45 | 10 | 10 | 10 | 10 |
| A | 125 | 125 | 125 | 500 |
| M | 50 | 50 | 75 | 250 |

A = 1-[(4-Amino-2-propyl-5-pyrimidinyl)methyl]-2-picoliniumchloridhydrochlorid (bekannt)

M = 2-(5-Ethyltetrahydro-5- (tetrahydro-3-methyl-5- (tetrahydro-6-hydroxy-6-(hydroxyethyl)- 3,5-dimethylpyran-2-yl)-2-furyl)-2-furyl)-9-hydroxy-β-methoxy-α, γ, 2,8-tetramethyl-1,6-dioxaspiro-(4,5)-decan-7-buttersäure (bekannt).

Herstellungsbeispiel 1

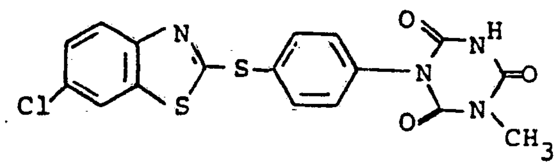

Zu einer Lösung von 4-(6-Chlorobenzothiazol-2-ylthio)-anilin (2,0 g) in Pyridin (20 ml) wurde unter Rühren Methylisocyanat (0,41 g) zugesetzt. Die Mischung wurde 12 h bei Raumtemperatur gerührt und zur Trockne eingedampft. Der Rückstand wurde im minimalen Volumen siedenden Ethanols gelöst und im Kühlschrank abgekühlt. Der resultierende feste Stoff wurde durch Filtration gesammelt und getrocknet, wodurch N-[4-(6-Chlorobenzothiazol-2-ylthio)phenyl]-N'-methylharnstoff (1,5 g) erhalten wurde, der bei 289–291 °C schmolz. Dieser Harnstoff (1,4 g) wurde in 1,4-Dioxan (50 ml) gelöst, und Chlorocarbonylisocyanat (0,47 g) wurde hinzugefügt. Die Mischung wurde 5 h zum Sieden erhitzt und zur Trockne eingedampft. Der feste Rückstand wurde im minimalen Volumen siedenden Ethanols gelöst und im Kühlschrank abgekühlt. Der resultierende feste Stoff wurde durch Filtration gesammelt und getrocknet, wodurch 1,3 g 1-[4-(6-Chlorobenzothiazol- 2-ylthio)phenyl]-3-methyl-1,3,5-triazin-2,4,6(1H, 3H, 5H)-trion erhalten wurde, das bei 232–233 °C schmolz.

Herstellungsbeispiel 2

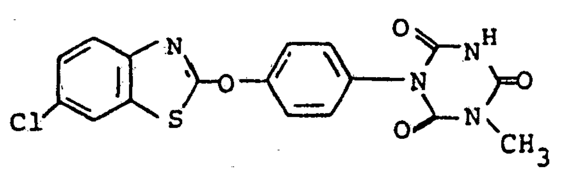

Zu einer Suspension von N-[4-(6-Chlorobenzothiazol- 2-yloxy)phenyl]- N'methylharnstoff (2,4 g) in 1,4-Dioxan (50 ml) wurde Chlorocarbonylisocyanat (0,8 g) hinzugefügt, und die Mischung wurde 5 h zum Sieden erhitzt. Die Mischung wurde unter vermindertem Druck eingedampft, und der Rückstand wurde im minimalen Volumen siedenden 1,4-Dioxans gelöst. Nach Zusatz von Ethanol (50 ml) wurde die klare Lösung im Kühlschrank abgekühlt. Der resultierende feste Stoff wurde durch Filtration gesammelt, mit kaltem Ethanol gewaschen und getrocknet, wodurch 2,1 g 1-[4-(6-Chlorobenzothiazol-2-yloxy)phenyl]- 3-methyl-1,3,5-triazin- 2,4,6(1H, 3H, 5H)-trion erhalten wurde, das bei 270–271 °C schmolz.

Herstellung von N-[4-(6-Chlorobenzothiazol-2-yloxy)-phenyl]-N'-methylharnstoff:

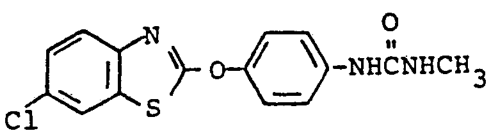

Zu einer Lösung von 4-Aminophenol (10,9 g) in N,N-Dimethylformamid (50 ml) wurde bei 10 °C bis 20 °C eine Lösung von Methylisocyanat (6 g) in Acetonitril (10 ml) zugetropft. Die Mischung wurde 12 h bei 25 °C bis 35 °C gerührt und unter vermindertem Druck eingedampft. Wasser (100 ml) wurde zu dem entstandenen Sirup hinzugefügt und die Mischung 30 min gerührt. Im Laufe des Rührens wurde die Mischung dick. Der feste Stoff wurde durch Filtration gesammelt, mit kaltem Wasser gewaschen und aus wässrigem Alkohol umkristallisiert, wonach 8,6 g N-(4-Hydroxyphenyl)-N'-methylharnstoff erhalten wurden, dessen Schmelzpunkt 169–171 °C betrug.

Zu einer Lösung des vorerwähnten Harnstoffs (3,23 g) in N,N-Dimethylformamid (60 ml) wurde wasserfreies Kaliumcarbonat (2,7 g) in einer Portion zugegeben. Eine Lösung von 2-Bromo-6-chlorobenzothiazol (4,7 g) in N,N-Dimethylformamid (20 ml) wurde zu der obigen Suspension bei 30 °C bis 40 °C zugetropft. Die Mischung wurde 1 h bei 50 °C, 1 h bei 80 °C und 4 h bei 120 °C bis 130 °C gerührt und dann abgekühlt. Die Reaktionsmischung wurde mit Wasser (260 ml) behandelt. Der entstandene feste Stoff wurde durch Filtration gesammelt, mit Wasser gewaschen und aus Ethanol umkristallisiert, wonach 4,9 g N-[4-(6-Chlorobenzothiazol-2-xyloxy)phenyl]-N'-methylharnstoff erhalten wurden, dessen Schmelzpunkt 225–228 °C betrug.

Die folgenden Verbindungen der Formel I wurden nach der gleichen Arbeitsweise wie derjenigen des Herstellungsbeispiels 1 hergestellt.

Tabelle 1

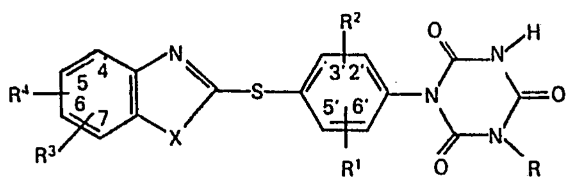

| Nr. | R | $R^1$ | $R^2$ | $R^3$ | $R^4$ | X | Schmp. °C |
|---|---|---|---|---|---|---|---|
| 3 | $CH_3CH_2-$ | H | H | H | H | S | 218 |
| 4 | $CH_3$ | H | H | 6–$CH_3$ | H | S | 200–202 |
| 5 | $CH_3$ | H | H | 6–$CH_3O-$ | H | S | 225–226 |
| 6 | $CH_3$ | H | H | 6–$CH_3CH_2O$ | H | S | 183–184 |
| 7 | $CH_3$ | H | H | 6–$CF_3$ | H | S | 207–215 |
| 8 | $CH_3$ | H | H | 5–Cl | H | S | 263–265 |
| 9 | $CH_3$ | H | H | 5–Cl | H | S | 205–215 |
| 10 | $CH_3CH_2$ | H | H | 5–Cl | H | S | 227–228 |
| 11 | $CH_3CH_2$ | H | H | 5–Cl | H | O | 218–221 |
| 12 | $CH_3$ | H | H | 6–F | H | O | 262–263 |
| 13 | $CH_3CH_2$ | H | H | 6–Cl | H | S | 195–205 |
| 14 | $CH_3$ | 3'–$CH_3$ | H | 6–Cl | H | S | 271–273 |
| 15 | $CH_3$ | 3'–Cl | H | 6–Cl | H | S | 271–273 |
| 16 | $CH_3$ | H | H | 6–Br | H | S | 231–232 |
| 17 | $CH_3$ | H | H | 6–I | H | S | 228–232 |
| 18 | $CH_3$ | H | H | 5–Cl | 6–Cl | S | 215–226 |
| 19 | $CH_3$ | H | H | 4–Cl | 6–Cl | S | 218–283 |
| 20 | $CH_3$ | H | H | 4–$CH_3$ | 6–Cl | S | 300–301 |
| 28 | $CH_3$ | H | H | 6–$OCF_3$ | H | S | |
| 29 | $CH_3$ | H | H | 6–$SCF_3$ | H | S | |

Die folgenden Verbindungen der Formel I wurden nach der gleichen Arbeitsweise wie derjenigen des Herstellungsbeispiels 2 hergestellt.

Tabelle 2

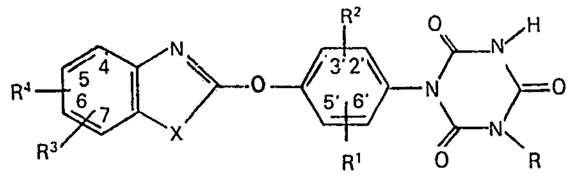

| Nr. | R | $R^1$ | $R^2$ | $R^3$ | $R^4$ | X | Schmp. °C |
|---|---|---|---|---|---|---|---|
| 21 | $CH_3$ | H | H | H | H | S | 256 |
| 22 | $CH_3$ | 3'–$CH_3$ | H | H | H | S | 289–296 |
| 23 | $CH_3CH_2$ | H | H | 6–Cl | H | S | 276–278 |
| 24 | $CH_3CH_2CH_2$ | H | H | 6–Cl | H | S | 227–232 |
| 25 | $CH_3$ | 3'–$CH_3$ | H | 6–Cl | H | S | 297–298 |
| 26 | $CH_3$ | 3'–Cl | 5'–Cl | 6–Cl | H | S | 280–282 |
| 27 | $CH_3$ | H | H | 6–Br | H | S | 279–281 |
| 30 | $CH_3$ | H | H | 6–$OC_2H_5$ | H | S | 227–229 |
| 31 | $CH_3$ | 3'–$CH_3$ | H | 6–$CF_3$ | H | S | 315–316 |
| 32 | $CH_3$ | 3'–Cl | 5'–Cl | 4–Cl | H | S | 228–230 |
| 33 | $CH_3$ | H | H | 5–Cl | H | S | 280–282 |
| 34 | $CH_3$ | 3'–$CH_3$ | H | 5–Cl | H | S | |
| 35 | $CH_3$ | 3'–Cl | 4'–Cl | 5–Cl | H | S | 216–219 |
| 36 | $CH_3$ | H | H | 6–F | H | S | 276–277 |
| 37 | $C_2H_5$ | 3'–Cl | 5'–Cl | 6–Cl | H | S | 244–246 |
| 38 | $CH_3$ | H | H | 6–Cl | H | O | |
| 39 | $CH_3$ | 3'–$CH_3$ | H | 6–Cl | H | O | |
| 40 | $CH_3$ | 3'–Cl | 5'–Cl | 6–Cl | H | O | |
| 41 | $CH_3$ | 3'–$CH_3$ | H | 6–Br | H | S | 299–302 |
| 42 | $CH_3$ | 3'–Cl | 5'–Cl | 6–Br | H | S | 288–293 |
| 43 | $CH_3$ | H | H | 6–J | H | S | 274–283 |
| 44 | $CH_3$ | H | H | 5–Cl | 6–Cl | S | 275–277 |
| 45 | $CH_3$ | 3'–$CH_3$ | H | 5–Cl | 6–Cl | S | 265–267 |
| 46 | $CH_3$ | 3'–$CH_3$ | 5'–$CH_3$ | 5–Cl | 6–Cl | S | 308–310 |
| 47 | $CH_3$ | H | H | 5–Cl | 6–Cl | O | |
| 48 | $CH_3$ | 3'–$CH_3$ | H | 5–Cl | 6–Cl | O | |
| 49 | $CH_3$ | 3'–$CH_3$ | 5'–$CH_3$ | 5–Cl | 6–Cl | O | |
| 50 | $CH_3$ | H | H | 6–$OCF_3$ | H | S | |
| 51 | $CH_3$ | 3'–$CH_3$ | H | 6–$OCF_3$ | H | S | |
| 52 | $CH_3$ | 3'–Cl | 5'–Cl | 6–$OCF_3$ | H | S | |
| 53 | $CH_3$ | H | H | 6–$SCF_3$ | H | S | |
| 54 | $CH_3$ | 3'–$CH_3$ | H | 6–$SCF_3$ | H | S | |
| 55 | $CH_3$ | 3'–Cl | 5'–Cl | 6–$SCF_3$ | H | S | |
| 56 | $CH_3$ | H | H | 7–Cl | H | S | 259–261 |
| 57 | $CH_3$ | 3'–Cl | 5'–Cl | 7–Cl | H | S | 287–290 |

Die folgenden Verbindungen der Formel II wurden nach der gleichen Arbeitsweise wie in Herstellungsbeispiel 2 für die Herstellung des N-[4-(6-Chlorbenzthiazol-2-yloxy)-phenyl]-N'-methylharnstoffs angegeben hergestellt.

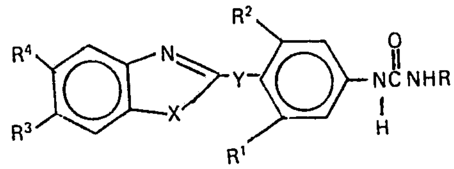

| $R^4$ | $R^3$ | X | Y | $R^2$ | $R^1$ | R | Schmelzpunkt |
|---|---|---|---|---|---|---|---|
| H | $CF_3$ | S | S | H | H | $CH_3$ | 210–220 |
| H | $CF_3$ | S | S | H | H | $CH_3$ | 224–229 |
| Cl | Cl | S | S | H | $CH_3$ | $CH_3$ | 197–210 |
| H | Cl | O | S | H | $CH_3$ | $CH_3$ | 243–244 |
| H | Cl | S | O | H | H | $CH_3$ | 225–258 |
| H | Cl | S | O | H | H | $C_2H_5$ | 215–217 |
| H | Cl | S | O | Cl | Cl | $CH_3$ | 240–242 |
| H | Cl | S | O | Cl | Cl | $C_2H_5$ | 208–211 |
| H | Br | S | O | H | $CH_3$ | $CH_3$ | 224–228 |
| H | I | S | O | H | H | $CH_3$ | 246–250 |
| H | $C_2H_5$ | S | O | H | H | $CH_3$ | 217–223 |
| Cl | H | S | O | H | H | $CH_3$ | 199–203 |
| Cl | H | S | O | Cl | Cl | $CH_3$ | 253–257 |
| Cl | Cl | S | O | H | H | $CH_3$ | 237–239 |
| Cl | Cl | S | O | H | $CH_3$ | $CH_3$ | 238–241 |
| Cl | Cl | S | O | Cl | Cl | $CH_3$ | 194–195 |
| H | Cl | O | O | H | H | $CH_3$ | 229–234 |
| H | Cl | O | O | H | $CH_3$ | $CH_3$ | 224–225 |
| H | $CF_3$ | S | O | H | $CH_3$ | $CH_3$ | 215–217 |
| H | $CF_3$ | S | O | H | H | $CH_3$ | 217–218 |
| H | $CF_3S$ | S | O | H | $CH_3$ | $CH_3$ | 209–211 |
| H | $CF_3O$ | S | O | H | H | $CH_3$ | 219–221 |
| H | $CF_3O$ | S | O | H | $CH_3$ | $CH_3$ | 192–193 |
| H | $CF_3O$ | S | O | Cl | Cl | $CH_3$ | 215–220 |

## Patentansprüche

1. 1-[-(Benzothia- oder -oxazol-2-ylthio- oder 2-yloxy)-phenyl]-1,3,5-triazin-2,4,6(1H, 3H, 5H)-trione der Formel I

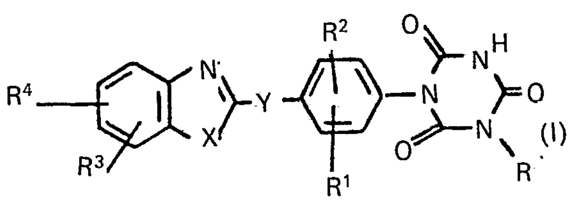

(I)

in der

R für $C_{1-4}$-Alkyl steht,

$R^1$ und $R^2$ gleich oder voneinander verschieden sind und für Wasserstoff, Halogen, $C_1$–$C_4$-Alkyl stehen,

$R^3$ für Halogen, $C_{1-4}$-Alkyl, $C_{1-4}$-Alkoxy, $C_{1-4}$-Alkylthio, $C_{1-4}$-Halogenalkyl, $C_{1-4}$-Halogenalkoxy, $C_{1-4}$-Alkylthio, $C_{1-4}$-Halogenalkylthio stehen,

$R^4$ für Wasserstoff oder Halogen steht,

X und Y gleich oder voneinander verschieden sind und für Schwefel oder Sauerstoff stehen

sowie Salze derselben.

2. Verbindungen der Formel I gemäss Anspruch 1, in welcher

R, $R^3$, $R^4$, X und Y die in Anspruch 1 angegebene Bedeutung haben und

$R^1$ und $R^2$ für Wasserstoff, Chlor oder $C_{1-4}$-Alkyl in 3'- und 5'-Stellung stehen.

3. Verbindungen der Formel I gemäss Anspruch 1, in welcher

R für Methyl, Ethyl oder Propyl steht,

$R^1$ und $R^2$ für Wasserstoff, Chlor oder Methyl in 3'- oder 5'-Stellung stehen,

$R^3$ für Fluor, Chlor, Brom, Jod, Methyl, Ethyl, Methoxy, Ethoxy, Methylthio, Trifluormethyl, Trifluormethoxy, Trifluormethylmercapto steht,

$R^4$ für Wasserstoff oder Chlor steht,

X und Y für 0 oder S stehen.

4. Verfahren zur Herstellung der Verbindungen der Formel I

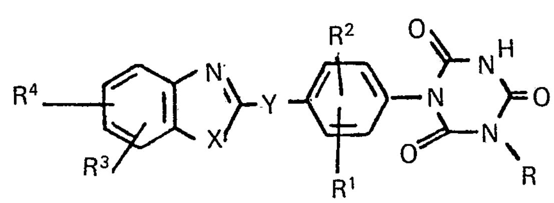

dadurch gekennzeichnet, dass man einen substituierten Harnstoff der Formel II

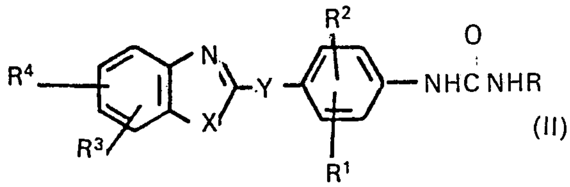

(II)

in der
R, $R^1$, $R^2$, $R^3$, $R^4$, X und Y die in Anspruch 1 angegebenen Bedeutungen haben, mit einem Carbonylisocyanat der Formel III

$$Z-\overset{O}{C}-N=C=O \qquad (III)$$

in der
Z für Halogen, Alkoxy oder Aryloxy steht, umsetzt und, im Falle der Salze eine Verbindung der Formel I mit einer Base umsetzt.

5. Arzneimittel, gekennzeichnet durch einen Gehalt an mindestens einem 1-[4-(Benzothia- oder -oxazol-2-ylthio- oder -2-yloxy)-phenyl]-1,3,5-triazin-2,4,6-(1H, 3H, 5H)-trion der Formel (I), gemäss Anspruch 1.

6. 1-[4-(Benzothia- oder oxazol-2-ylthio- oder -2-yloxy)-phenyl]-1,3,5-triazin-2,4,6-(1H, 3H, 5H)-trion der Formel (I) gemäss Anspruch 1 zur Bekämpfung von human- und tierpathogenen Coccidien.

7. Verfahren zur Herstellung von Arzneimitteln, dadurch gekennzeichnet, dass man 1-[4-(Benzothia- oder -oxazol-2-ylthio- oder -2-yloxy)-phenyl]-1,3,5-triazin-2,4,6(1H, 3H, 5H)-trion der Formel (I) gemäss Anspruch 1 mit Streckmitteln und/oder oberflächenaktiven Mitteln vermischt.

8. Verwendung von Verbindungen der allgemeinen Formel (I) in Anspruch 1 als Zusatz zu Futter- und Nahrungsmitteln in der Tierernährung.

9. Tiernahrung und in der Tierhaltung verwendete Trinkwasser, enthaltend Verbindungen der allgemeinen Formel (I) in Anspruch 1.

10. Praemixe für die Tiernahrung und/oder in der Tierhaltung verwendete Trinkwasser, enthaltend Verbindungen der allgemeinen Formel (I) in Anspruch 1.

**Revendications**

1. 1-[4-(benzothia- ou -oxazol-2-ylthio- ou -2-yloxy)phényl]-1,3,5-triazine-2,4,6(1H, 3H, 5H)-triones de formule I:

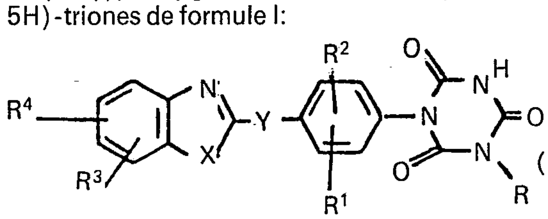

(I)

dans laquelle
R représente un groupe alkyle en $C_1$–$C_4$,
$R^1$ et $R^2$ sont identiques ou différents l'un de l'autre et représentent chacun un atome d'hydrogène, un atome d'halogène ou un groupe alkyle en $C_1$–$C_4$,
$R^3$ représente un atome d'halogène, un groupe alkyle en $C_1$–$C_4$, un groupe alcoxy en $C_1$–$C_4$, un groupe alkyl (en $C_1$–$C_4$)thio, un groupe halogénalkyle en $C_1$–$C_4$, un groupe halogénalcoxy en $C_1$–$C_4$, un groupe alkyl (en $C_1$–$C_4$)thio ou un groupe halogénalkyl(en $C_1$–$C_4$)thio,
$R^4$ représente un atome d'hydrogène ou un atome d'halogène,
X et Y sont identiques ou différents l'un de l'autre et représentent chacun un atome de soufre ou un atome d'oxygène, ainsi que leurs sels.

2. Composés de formule I selon la revendication 1, formule dans laquelle:
R, $R^3$, $R^4$, X et Y ont les significations indiquées dans la revendication 1, et
$R^1$ et $R^2$ représentent chacun un atome d'hydrogène, un atome de chlore ou un groupe alkyle en $C_1$–$C_4$ dans les positions 3' et 5'.

3. Composés de formule I selon la revendication 1, formule dans laquelle:
R représente le groupe méthyle, le groupe éthyle ou le groupe propyle,
$R^1$ et $R^2$ représentent chacun l'atome d'hydrogène, l'atome de chlore ou le groupe méthyle en position 3' ou 5',
$R^3$ représente l'atome de fluor, l'atome de chlore, l'atome de brome, l'atome d'iode, le groupe méthyle, le groupe éthyle, le groupe méthoxy, le groupe éthoxy, le groupe méthylthio, le groupe trifluorométhyle, le groupe trifluorométhoxy ou le groupe trifluorométhylmercapto,
$R^4$ représente l'atome d'hydrogène ou l'atome de chlore,
X et Y représentent 0 ou S.

4. Procédé de préparation des composés de formule I:

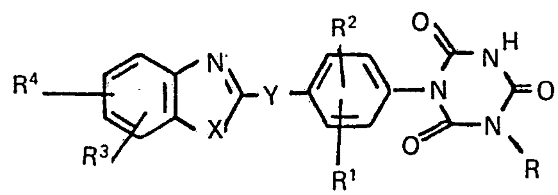

caractérisé en ce qu'on fait réagir une urée substituée de formule II:

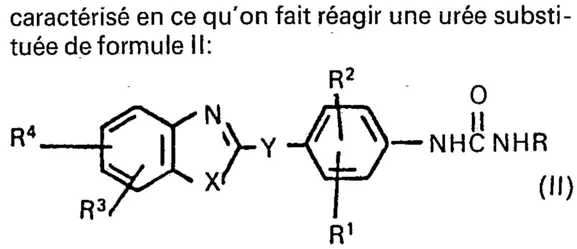

(II)

dans laquelle
R, $R^1$, $R^2$, $R^3$, $R^4$, X et Y ont les significations indiquées dans la revendication 1, avec un isocyanate de carbonyle de formule III:

$$Z-\overset{O}{\overset{\|}{C}}-N=C=O \qquad (III)$$

dans laquelle

Z représente un atome d'halogène, un groupe alcoxy ou un groupe aryloxy,

et, dans le cas des sels, on fait réagir un composé de formule I avec une base.

5. Médicament, caractérisé en ce qu'il contient au moins une 1-[4-(benzothia- ou -oxazol-2-ylthio- ou -2-yloxy)-phényl]-1,3,5-triazine-2,4,6(1H, 3H, 5H)-trione de formule (I) selon la revendication 1.

6. 1-[4-(benzothia- ou -oxazol-2-ylthio- ou -2-yloxy)-phényl]-1,3,5-triazine-2,4,6-(1H, 3H, 5H)-trione de formule (I) selon la revendication 1 pour combattre les coccidies pathogènes chez l'homme et l'animal.

7. Procédé de préparation de médicaments, caractérisé en ce qu'on mélange une 1-[4-(benzothia- ou -oxazol-2-ylthio- ou -2-yloxy)-phényl]-1,3,5-triazine-2,4,6-(1H, 3H, 5H)-trione de formule (I) selon la revendication 1 avec des diluants et/ou des agents tensio-actifs.

8. Utilisation de composés de formule générale (I) selon la revendication 1, comme additifs à des aliments pour animaux et des produits alimentaires dans l'alimentation des animaux.

9. Alimentation des animaux et eau de boisson utilisée dans l'alimentation des animaux, contenant des composés de formule générale (I) selon la revendication 1.

10. Prémélanges pour l'alimentation des animaux et/ou dans l'eau de boisson utilisée pour l'alimentation des animaux, contenant des composés de formule générale (I) selon la revendication 1.

## Claims

1. 1-[-(Benzothia- or -oxazol-2-ylthio- or 2-yloxy)-phenyl]-1,3,5-triazine-2,4,6 (1H, 3H, 5H)-triones of the formula I

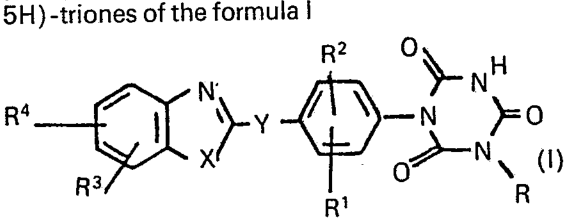

(I)

in which

R represents $C_1$–$C_4$-alkyl,

$R^1$ and $R^2$ are identical or different and represent hydrogen, halogen or $C_1$–$C_4$-alkyl,

$R^3$ represents halogen, $C_1$–$C_4$-alkyl, $C_1$–$C_4$-alkoxy, $C_1$–$C_4$-alkylthio, $C_1$–$C_4$-halogenoalkyl, $C_1$–$C_4$-halogenoalkoxy, $C_1$–$C_4$-alkylthio or $C_1$–$C_4$-halogenoalkylthio,

$R^4$ represents hydrogen or halogen,

X and Y are identical or different and represent sulphur or oxygen, and salts thereof.

2. Compounds of the formule I according to Claim 1, in which

R, $R^3$, $R^4$, X and Y have the meaning given in Claim 1 and

$R^1$ and $R^2$ represent hydrogen, chlorine or $C_{1-4}$-alkyl in the 3'- and 5'-position.

3. Compounds of the formule I according to Claim 1, in which

R represents methyl, ethyl or propyl,

$R^1$ and $R^2$ represent hydrogen, chlorine or methyl in the 3'- or 5'-position,

$R^3$ represents fluorine, chlorine, bromine, iodine, methyl, ethyl, methoxy, ethoxy, methylthio, trifluoromethyl, trifluoromethoxy or trifluoromethylmercapto,

$R^4$ represents hydrogen or chlorine and

X and Y represent 0 or S.

4. Process for the preparation of the compounds of the formula I

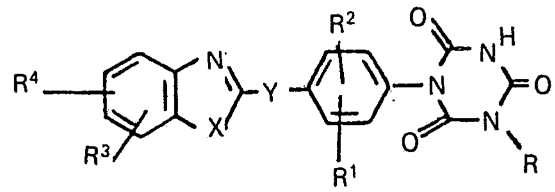

characterized in that a substituted urea of the formule II

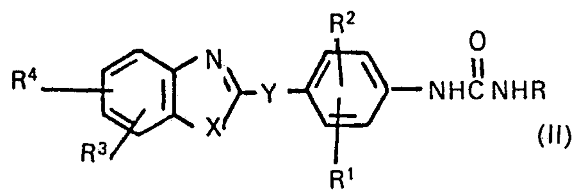

(II)

in which

R, $R^1$, $R^2$, $R^3$, $R^4$, X and Y have the meanings given in Claim 1, is reacted with a carbonyl isocyanate of the formula III

$$Z-\overset{O}{\overset{\|}{C}}-N=C=O \qquad (III)$$

in which

Z represents halogen, alkoxy or aryloxy, and, in the case of the salts, a compound of the formula I is reacted with a base.

5. Medicaments, characterized in that they contain at least 1-[4-(benzothia- or -oxazol-2-ylthio- or -2-yloxy)-phenyl]-1,3,5-triazine-2,4,6-(1H, 3H, 5H)-trione of the formula (I) according to Claim 1.

6. Use of 1-[4-(benzothia- or -oxazol-2-ylthio- or -2-yloxy)-phenyl]-1,3,5-triazine-2,4,6-(1H, 3H, 5H)-trione of the formula (I) according to Claim 1 for combating Coccidia which are pathogenic to humans and animals.

7. Process for the preparation of medicaments, characterized in that 1-[4-(benzothia- or -oxazol-2-ylthio- or -2-yloxy)-phenyl]-1,3,5-triazine-2,4,6(1H, 3H, 5H)-trione of the formule (I) according to Claim 1 is mixed with extenders and/or surface-active agents.

8. Use of compounds of the general formula (I) in Claim 1 as an additive to feedstuffs and foodstuffs in animal nutrition.

9. Animal food, and drinking water used in the keeping of animals, containing compounds of the general formula (I) in Claim 1.

10. Premixes for animal food and/or for drinking water used in the keeping of animals, containing compounds of the general formula (I) in Claim 1.